(19) Europäisches Patentamt / European Patent Office / Office européen des brevets

(11) **EP 2 843 214 B1**

(12) **EUROPÄISCHE PATENTSCHRIFT**

(45) Veröffentlichungstag und Bekanntmachung des Hinweises auf die Patenterteilung:
**23.06.2021 Patentblatt 2021/25**

(51) Int Cl.:
*H01M 8/04089* (2016.01)    *F23N 5/00* (2006.01)
*H01M 8/0438* (2016.01)    *H01M 8/0444* (2016.01)
*H01M 8/124* (2016.01)    *G01F 1/68* (2006.01)
*G01N 33/22* (2006.01)    *H01M 8/04746* (2016.01)

(21) Anmeldenummer: **14169594.0**

(22) Anmeldetag: **23.05.2014**

(54) **Verfahren, Sensor und Regelvorrichtung zur Regelung gasbetriebener Energiewandleranlagen**

Method, sensor and control device for controlling gas-powered energy conversion systems

Procédé, capteur et dispositif de réglage d'installations de conversion d'énergie fonctionnant au gaz

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **29.05.2013 EP 13169741**

(43) Veröffentlichungstag der Anmeldung:
**04.03.2015 Patentblatt 2015/10**

(73) Patentinhaber:
- **MEMS AG**
  **5413 Birmenstorf (CH)**
- **Hexis AG**
  **8404 Winterthur (CH)**

(72) Erfinder:
- **Denzler, Roland**
  **8484 Weisslingen (CH)**
- **Prêtre, Philippe**
  **5405 Dättwil (CH)**
- **Kempe, Andreas**
  **8049 Zürich (CH)**

(74) Vertreter: **Intellectual Property Services GmbH**
**Langfeldstrasse 88**
**8500 Frauenfeld (CH)**

(56) Entgegenhaltungen:
EP-A1- 1 265 068    EP-A1- 2 015 056
EP-A1- 2 574 918

- **None**

**EP 2 843 214 B1**

**Beschreibung**

Die Erfindung bezieht sich auf ein Verfahren zur Regelung gasbetriebener und insbesondere erdgasbetriebener Energiewandleranlagen gemäss Oberbegriff von Anspruch 1 sowie auf einen thermischen Sensor und auf eine Regelvorrichtung zur Regelung gasbetriebener und insbesondere erdgasbetriebener Energiewandleranlagen gemäss Oberbegriff der Ansprüche 8 und 11.

Unter Energiewandleranlagen fallen z.B. Wärme-Kraft-Koppelungsanlagen (WKK), Brennstoffzellenanlagen, insbesondere Brennstoffzellenanlagen mit Hochtemperatur-Brennstoffzellen des SOFC-Typs ("Solid Oxid Fuel Cell"), die bei 700 °C bis 960 °C betrieben werden, Heizgeräte resp. Gasthermen, Warmwassererzeuger, gasbetriebene Wärmepumpen und Stromerzeugungsanlagen sowie Kombinationen davon. All diesen Anlagen ist eigen, dass eine effiziente und emissionsarme Energieumsetzung davon abhängt, ob dem Brenngas die prozessspezifisch optimale Menge an Sauerstoffträger zugefügt wird. Als Prozess kann dies sowohl die Reformierung, partielle Oxidation wie auch die komplette Oxidation des Brenngases betreffen. Dient beispielsweise bei einem Verbrennungsmotor der Luftsauerstoff als Sauerstoffträger zur kompletten Oxidation, kann es sich beim Umsetzungsprozess (Reformierung, partielle Oxidation) bei einer Brennstoffzelle auch um Luft, Wasser oder Kohlendioxid als Sauerstoffträger handeln, oder um ein Gemisch der drei genanten Sauerstoffträger wie etwa bei der Anodengasrezirkulation.

Die optimale Menge an Sauerstoffträger hängt von der Zusammensetzung des Brenngases, d.h. von der Brenngasqualität ab. Je nach Region und Zulieferer können erhebliche Schwankungen in der Brenngasqualität auftreten, auf die nur mit deren Kenntnis reagiert werden kann. Beim Umsetzungsprozess (Reformierung, partielle Oxidation) bedeutet ein Zuviel an Sauerstoffträger einen ineffizienten, zu heiss laufenden Prozess, wohingegen zu wenig Sauerstoffträger mit einem verringerten Brenngasumsatz bis hin zur Verrussung einher geht. Letztendlich führen beide Situationen zur Prozessinstabilität oder zur Schädigung bzw. zur Verkürzung der Lebensdauer der Anlage.

Die Aufgabe der Zuführung von Sauerstoffträger zum Brenngas übernimmt in der Regel eine Brenngas-Sauerstoffträger-Verbundregelung, die im Fall von Luft als Sauerstoffträger als Brenngas-Luft-Verbundregelung bezeichnet wird. Ohne Kenntnis der Brenngasqualität ist die Dosierung der idealen Mengen jedoch nicht möglich. Ein fixes Mischungsverhältnis muss deshalb so gewählt werden, dass die Schwankungen in der Brenngasqualität unter keinen Umständen zu einer Schädigung der Anlage führen können, womit man bei "guter" Brenngasqualität an Effizienz verliert, da eine Sicherheitsmarge zum optimalen Wert eingehalten werden muss.

Als Lambdawert λ oder, im Fall von Luft als Sauerstoffträger, als Luftzahl bezeichnet man das Verhältnis Sauerstoffträger zu Brenngas im Vergleich zu einem stöchiometrischen Gemisch, bei welchem die theoretisch benötigte Sauerstoffträgermenge für eine vollständige Verbrennung vorhanden, d.h. λ=1 ist. Bezeichnet man das ideale Verhältnis aus Sauerstoffträger- zu Brenngasmenge für den angestrebten Oxidationsprozess mit $\lambda_{id}$, folgt für das ideale Mischverhältnis $r_{id}$

$$r_{id} = L_{min} \cdot \lambda_{id} \, , \qquad\qquad (1)$$

worin $L_{min}$ die minimale Sauerstoffträgermenge für eine λ=1 Verbrennung bedeutet. Im Fall von Luft als Sauerstoffträger wird $L_{min}$ auch als minimale Luftmenge bezeichnet.

Aus WO 2006/061228 A1 ist ein Verfahren zur Bestimmung der Luftzahl bei einem Brenner für ein Brennstoffzellenheizgerät bekannt, bei dem nebst der in der Brennertechnik verwendeten, bekannten Flammionisationsstrommessung zusätzlich weitere Zustandsgrössen zur Abschätzung der Brenngaszusammensetzung und folglich der Luftzahl bestimmt werden. Allerdings basieren die Zustandsgrössen auf den im Gerät umgesetzten Gasmengen, über deren Bestimmung a priori keine Aussage getroffen wird.

Aus EP 1 923 634 A1 ist ein Verfahren zur Regelung eines Brenngas-LuftGemisches eines Heizgeräts mittels einer Ausgangstemperaturmessung bekannt, wobei bei Vorgabe einer Brennerbelastung der notwendige Brenngasvolumen- oder -massenstrom und unter Berücksichtigung der Verbrennungsluftzahl λ der Verbrennungsluftvolumen -oder -massenstrom sowie aus einem Kennfeld oder einer Funktion die belastungsabhängige Brenner- oder Flammensolltemperatur bestimmt werden. Es findet sich allerdings kein Hinweis darauf, woher bei einer solchen Regelung im Falle wechselnder Brenngasqualitäten die notwendigen Brenngas- und Luftvolumen- bzw. -massenströme bekannt sind.

Aus der EP 2 574 918 A1 ist ein Verfahren zur Bestimmung physikalischer Gaseigenschaften von Gasgemischen zur Korrelation brenntechnisch relevanter Grössen der Gasgemische bekannt. Hierbei wird mittels eines ersten Sensors unter anderem die Dichte, die Wärmekapazität, der Massen- oder Volumenfluss eines Gases bestimmt. Zudem wird mit einem zweiten, mikrothermischen Sensor die Wärmeleitfähigkeit des Gases separat gemessen.

Im Folgenden wird von der das thermische Sensorsystem beschreibenden, eindimensionalen Wärmeleitungsgleichung (Kerson Huang: Statistical Mechanics, 2.Auflage, John Wiley & Sons, New York 1987, ISBN 0-471-85913-3) ausgegangen.

$$\frac{c_p}{\kappa} \cdot \rho \, \mathrm{v_x} \cdot \frac{d}{dx} T = \nabla^2 T + \frac{1}{\kappa} \Theta \,, \qquad (2)$$

wobei

$v_x$    die Komponente der mittleren Fliessgeschwindigkeit (Geschwindigkeitsvektor) $\vec{v}$ in x-Richtung, d. h. entlang des Gasstromes,

$T$    die Temperatur,

$$\frac{d}{dx} T$$

     der Temperaturgradient,

$c_p$    die Wärmekapazität des Gases bei konstantem Druck,

$\rho$    die Dichte,

$\kappa$    die Wärmeleitfähigkeit des Gases,

$\nabla^2 T$    der Laplace-Operator, angewandt auf die Temperatur T, wobei

$$\nabla^2 = \left(\frac{d}{d_x}\right)^2 + \left(\frac{d}{dy}\right)^2 + \left(\frac{d}{dz}\right)^2$$

bedeuten. Da das Gas (Gasstrom) nur in x-Richtung fliesst, werden die Komponenten $v_y$ bzw. $v_z$ in y-Richtung bzw. in z-Richtung der mittleren Fliessgeschwindigkeit $\vec{v}$ zu Null angenommen. $\Theta$ mit der Einheit Watt/m$^3$ beschreibt den Quellterm des Heizelements. Der Quellterm rührt bei der thermischen Methode vom Heizdraht eines Hitzdrahtanemometers her, der Wärmeenergie in das System speist. Die Umrechnung von mittlerer Fliessgeschwindigkeit in Volumenfluss ist dabei durch die (Querschnitts-)Geometrie der zuführenden Gasleitung gegeben.

Wird nun Gleichung (1) auf einen mit einem Kalibriergas (CalGas) kalibrierten Brenngas-Sauerstoffträger-Verbund bezogen und letzterer mit einer anderen Brenngasmischung (Gas) beaufschlagt, stellt sich von selbst der folgende Lambdawert, $\lambda_{actual}$, ein:

$$\lambda_{actual} = r_{id\_CalGas} \cdot (1 + \Delta(\%)) / L_{min\_Gas} \,, \qquad (3)$$

wobei sich der Brenngasflussfehler $\Delta$ auf den Unterschied zwischen dem vom Gerät eingestellten Sollwert zum effektiv vorhandenen Brenngasfluss bezieht. Wird also zuviel Durchfluss angezeigt, fliesst effektiv weniger Brenngas, was zu einer Erhöhung des Sauerstoffträger-/Brenngasverhältnisses r führt.

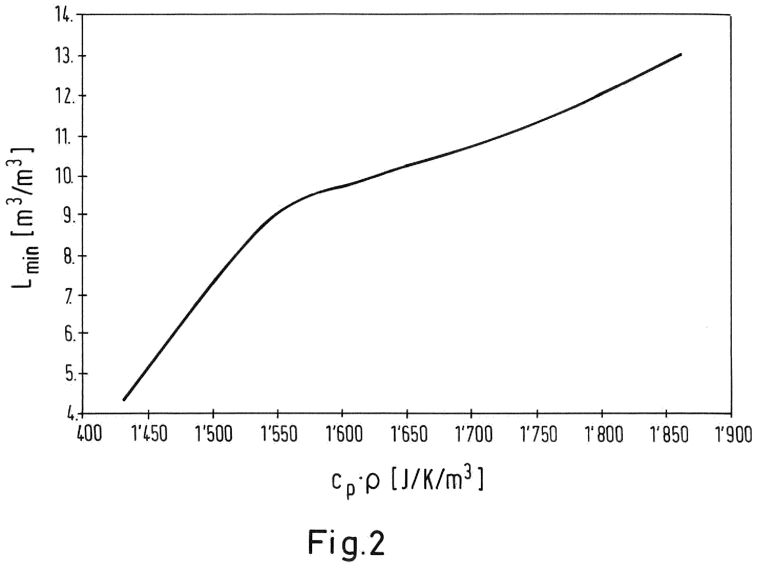

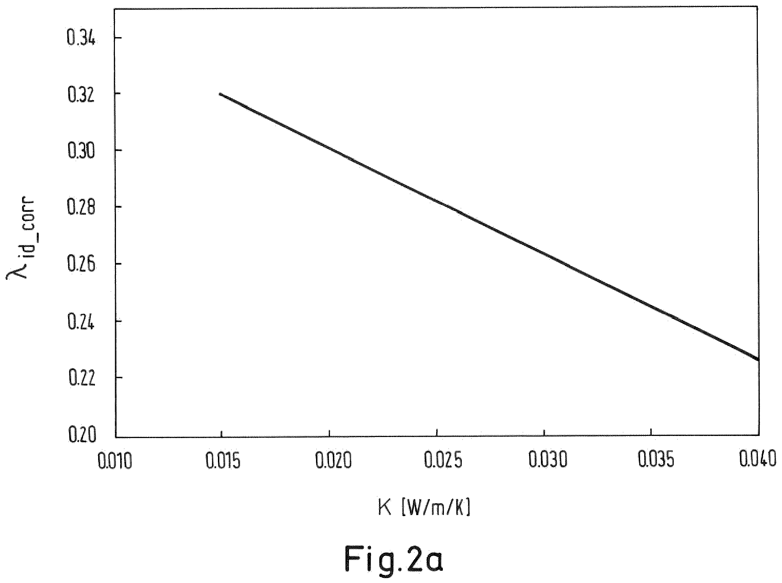

Basierend auf Gleichung (2) des thermischen Sensorsystems kann andererseits die Bestimmung des idealen Lambdawertes, $\lambda_{id}$, und des minimalen Sauerstoffträgerbedarfs, $L_{min}$, mittels Korrelation aus einer der Grössen $\kappa$, $C_p$ und/oder $\rho$ oder aus einer Kombination dieser Grössen erfolgen, wie beispielsweise in Fig. 2 für $L_{min}$ als Funktion des Produkts aus Wärmekapazität und Dichte $c_p \cdot \rho$ oder in Fig. 2a für $\lambda_{id}$ als Funktion der Wärmeleitfähigkeit $\kappa$ für das Gebiet der Erdgase gezeigt ist. Dann liest sich in der zur Gleichung (3) analogen Gleichung der sich einstellende Lambdawert $\lambda_{actual}$ als

$$\lambda_{actual} = r_{id\_corr} \cdot (1 + \Delta(\%)) / L_{min\_Gas} \,, \qquad (4)$$

mit der gleichen Auswirkung eines Fehlers $\Delta$ in der Flussbestimmung wie in Gleichung (3), wobei $r_{id\_corr} = L_{min\_corr} \cdot \lambda_{id\_corr}$, analog zu Gleichung (1). Je nach gewählter Korrelation können sich Fehler im korrelierten Lambdawert, $\lambda_{id\_corr}$, und im korrelierten minimalen Sauerstoffträgerbedarf, $L_{min\_corr}$, zusammen mit dem Flussfehler dämpfend oder verstärkend auf die Abweichung des aktuellen zum idealen Lambdawert, $\lambda_{actual} - \lambda_{id}$, für die gerade anliegende Brenngasqualität auswirken, was einen positiven oder negativen Effekt auf die Effizienz und die Betriebssicherheit der Energiewandleranlage hat.

Der Erfindung liegt daher die Aufgabe zu Grunde, ein Verfahren sowie einen thermischen Sensor und eine Regelvorrichtung zur Brenngas-Sauerstoffträger-Verbundregelung gasbetriebener Energiewandleranlagen anzugeben, die eine energetisch effizienten Umsetzung des zugeführten Brenngases und einen stabilen Betrieb bei wechselnder Brenngasqualität ermöglichen.

Erfindungsgemäss wird die Aufgabe bezüglich des Verfahrens gelöst durch ein Verfahren mit den Merkmalen nach Anspruch 1. Bezüglich des Sensors wird die Aufgabe gelöst durch einen Sensor mit den Merkmalen nach Anspruch 8 und bezüglich der Regelvorrichtung durch eine Regelvorrichtung mit den Merkmalen nach Anspruch 11.

In dem Verfahren gemäss vorliegender Erfindung zur Brenngas-Sauerstoffträger-Verbundregelung einer gasbetriebenen Energiewandleranlage wird der Massen- oder Volumendurchfluss des Brenngases und/oder Sauerstoffträgers erfasst, um das Mischungsverhältnis r von Brenngas und Sauerstoffträger zu regeln. Das Verfahren zeichnet sich dadurch aus, dass mit einem mikrothermischen Sensor mindestens zwei physikalische Kenngrössen des Brenngases ermittelt werden, wobei die erste Kenngrösse den Massen- und/oder Volumendurchfluss des Brenngases und die zweite Kenngrösse die Wärmeleitfähigkeit und/oder Wärmekapazität umfassen, und dass aus den physikalischen Kenngrössen ein vom Brenngas oder von der Zusammensetzung des Brenngases abhängiger Sollwert für das Mischungsverhältnis bestimmt wird, der zum Regeln des Mischungsverhältnisses verwendet wird. Mit anderen Worten werden die mindestens zwei physikalischen Kenngrössen des Brenngases mit demselben mikrothermischen Sensor ermittelt.

Es wurde nun ausgehend von den Gleichungen (3) bzw. (4) gefunden, dass - im Gegensatz zum Vorurteil im Stand der Technik wie beispielsweise in der EP 2 574 918 A1 ausgeführt - das Verhältnis aus den beiden Kenngrössen mit einem mikrothermischen Sensor zuverlässig bestimmbar ist; somit ist kein anderer Sensortyp und damit kein separater Sensor für die erste Kenngrösse notwendig, da sich die Auswirkung des Fehlers Δ entsprechend kompensieren lässt wie zu den Gleichungen (3) und (4) vorstehend dargelegt. Somit kann die Differenz zwischen dem sich einstellenden Lambdawert und dem idealen Lambdawert des bei wechselnder Brenngasqualität zugrunde liegenden Brenngases klein gehalten werden, um einen anwendungsspezifisch günstigen Prozesspunkt zu erzielen; zudem kann sichergestellt werden, dass die Differenz nie negativ wird, um die Betriebssicherheit nicht zu gefährden.

Die mindestens zwei physikalischen Kenngrössen, die mit demselben mikrothermischen Sensor ermittelt wurden, werden vorteilhafterweise sowohl zur Brenngasqualitäts- als auch zur Brenngasflussbestimmung benützt.

Dies hat den Vorteil, dass mit demselben mikrothermischen Sensor die zwei Kenngrössen bestimmbar sind, was die Vorrichtung kostengünstiger macht, da lediglich ein Sensor benötigt wird. Zudem muss gegebenenfalls lediglich ein Sensor kalibriert werden.

In einer vorteilhaften Ausführungsform des Verfahrens werden die zwei physikalischen Kenngrössen, die mit demselben mikrothermischen Sensor ermittelt wurden, herangezogen, um einerseits mittels Korrelation den idealen Lambdawert $\lambda_{id}$ und den minimalen Sauerstoffträgerbedarf $L_{min}$ zu bestimmen und andererseits den Massen- und/oder Volumenfluss des Brenngases.

Die Bestimmung der physikalischen Kenngrössen und der daraus korrelierten Grössen können beispielsweise auf ein Kalibriergas bezogen werden, wobei die Abweichung des kalibrierten Lambdawertes $\lambda_{CalGas}$ zum idealen Lambdawert $\lambda_{id}$ durch den damit gekoppelten Durchflussbestimmungsfehler kompensiert wird.

Die physikalischen Kenngrössen und die daraus korrelierten Grössen können aber auch für ein beliebiges Brenngas in Teilen neu bestimmt und die restlichen vom Kalibriergas übernommen werden, wobei die Abweichung des sich einstellenden Lambdawertes $\lambda_{actual}$ zum idealen Lambdawert $\lambda_{id}$ durch den damit gekoppelten Durchflussbestimmungsfehler zumindest teilweise kompensiert wird. Dabei kann beispielsweise die Wärmekapazität und/oder Wärmeleitfähigkeit vom Kalibriergas übernommen werden.

In einer weiteren vorteilhaften Ausführungsform des Verfahrens werden die physikalischen Kenngrössen und die daraus korrelierten Grössen für ein beliebiges Brenngas in Gänze vom Kalibriergas übernommen, wobei die Abweichung des sich einstellenden Lambdawertes $\lambda_{actual}$ zum idealen Lambdawert $\lambda_{id}$ durch den damit gekoppelten Durchflussbestimmungsfehler zumindest teilweise kompensiert wird.

Unabhängig von der Ausführungsform des Verfahrens kann die Energiewandleranlage eine Brennstoffzellenanlage, insbesondere Brennstoffzellenanlage mit Hochtemperatur-Brennstoffzellen des SOFC-Typs, eine Wärme-Kraft-Koppelungsanlage, ein Gasmotor, ein Heizgerät resp. eine Gastherme, eine gasbetriebene Wärmepumpe, ein Warmwassererzeuger, und/oder eine gasbetriebene Stromerzeugungsanlage oder eine Kombination davon sein.

Der Sensor gemäss vorliegender Erfindung zur Brenngas-Sauerstoffträger-Verbundregelung einer gasbetriebenen Energiewandleranlage, ist mit einer Auswerte- und Regeleinheit, die zur Ausführung eines Verfahrens zur Brenngas-Sauerstoffträger-Verbundregelung gemäß oben stehender Beschreibung eingerichtet, und mit einem mikrothermischen Hitzdrahtanemometer ausgestattet, welches mit einem Brenngas beaufschlagbar ist, um mindestens zwei physikalische Kenngrössen des Brenngases zu ermitteln, zum Beispiel als erste physikalische Kenngrösse die Wärmeleitfähigkeit und/oder Wärmekapazität und als zweite physikalische Kenngrösse zum Beispiel den Massen- und/oder Volumenfluss eines mikrothermischen Verfahrens, und um aus den physikalischen Kenngrössen einen vom Brenngas oder von der Zusammensetzung des Brenngases abhängigen Sollwert für das Mischungsverhältnis zu bestimmen und damit die entsprechenden Brenngas- und/oder Sauerstoffträgermengen zu regeln.

In einer weiteren vorteilhaften Ausführungsform ist das mindestens eine mikrothermische Hitzdrahtanemometer als integriertes CMOS-Hitzdrahtanemometer ausgeführt.

Unabhängig von der Ausführungsform kann der Sensor eingerichtet sein, um die Brenngasmenge und/oder Sauerstoffträgermenge mittels traditionellen thermischen Massenflussmessern bzw. -reglern zu regeln.

Weiter umfasst die Erfindung eine Regelvorrichtung zur Brenngas-Sauerstoffträger-Verbundregelung einer gasbetriebenen Energiewandleranlage, wobei die Regelvorrichtung einen Sensor zur Brenngas-Sauerstoffträger-Verbundregelung gemäß oben stehender Beschreibung und mindestens ein Ventil enthält, um die Brenngas- bzw. Sauerstoffträgermenge oder -mengen zu regeln.

In einer vorteilhaften Ausführungsform der Regelvorrichtung zur Brenngas-Sauerstoffträger-Verbundregelung einer gasbetriebenen Energiewandleranlage ist das Ventil als Mischventil ausgebildet.

Die Erfindung umfasst zudem eine Brennstoffzellenanlage, insbesondere eine Brennstoffzellenanlage mit Hochtemperatur-Brennstoffzellen des SOFC-Typs, mit einer Regelvorrichtung zur Brenngas-Sauerstoffträger-Verbundregelung gemäss oben stehender Beschreibung.

Die Idee der Erfindung ist also die Angabe eines Verfahrens, eines Sensors und einer Regelvorrichtung zur energetisch effizienten Umsetzung des zugeführten Brenngases, wie beispielsweise Erdgas, und zur Sicherstellung der Stabilität und Lebensdauer gasbetriebener Energiewandleranlage mit Brenngas-Sauerstoffträger-Verbundregelung, wobei mit einem thermischen Sensor einerseits physikalische Kenngrössen wie beispielsweise der ideale Lambdawerte $\lambda_{id}$ und der minimale Sauerstoffträgerbedarf $L_{min}$ bestimmt werden, und andererseits mit demselben Sensor die Durchflussmenge im Brenngas-Sauerstoffträger-Verbund bei wechselnden Brenngasqualitäten gemessen wird, um den gasbetriebenen Energiewandleranlage auf das ideale Mischverhältnis $r_{id}$ zu regeln.

Das Verfahren, der thermische Sensor und die Regelvorrichtung zur Brenngas-Sauerstoffträger-Verbundregelung gasbetriebener Energiewandleranlagen gemäss vorliegender Erfindung haben den Vorteil, dass sie vergleichsweise einfach und kostengünstig umgesetzt bzw. hergestellt werden können, und dass die Differenz zwischen dem sich einstellenden Lambdawert und dem idealen Lambdawert des bei wechselnder Brenngasqualität zugrunde liegenden Brenngases einerseits klein gehalten werden kann, um einen anwendungsspezifisch günstigen Prozesspunkt zu erzielen, und andererseits nie negativ wird, um die Betriebssicherheit nicht zu gefährden.

Weitere Vorteile sind aus der nachstehenden Beschreibung ersichtlich. Vorteilhafte Ausgestaltungen der Erfindung finden sich in den Unteransprüchen.

Die Erfindung wird nachstehend anhand der Zeichnungen näher erläutert. Es zeigen:

Fig. 1 einen Sensorkopf eines integrierten CMOS-Anemometers,

Fig. 2 die Korrelation zwischen minimalem Sauerstoffträgerbedarf und dem Produkt aus Wärmekapazität und Dichte des Brenngases,

Fig. 2a die Korrelation zwischen idealem Verhältnis aus Sauerstoffträger- zu Brenngasmenge und der Wärmeleitfähigkeit des Brenngases,

Fig. 3 die Abweichung des sich einstellenden Lambdawertes zum idealen Lambdawert als Funktion des Sauerstoffträgerbedarfs bei Unwissenheit der Brenngasqualität, aber fehlerlosen Gasflussbestimmung im Falle einer SOFC-Brennstoffzelle mit partieller Oxidation im Vergleich zu einem Kalibriergas,

Fig. 4 die Abweichung des sich einstellenden Lambdawertes zum idealen Lambdawert als Funktion des Flussfehlers für einen herkömmlichen Massenflussmesser bzw. -regler im Falle einer SOFC-Brennstoffzelle mit partieller Oxidation im Vergleich zu einem Kalibriergas,

Fig. 5 die Abweichung des sich einstellenden Lambdawertes zum idealen Lambdawert als Funktion des Flussfehlers für einen mikrothermischen Sensor im Falle einer SOFC-Brennstoffzelle mit partieller Oxidation im Vergleich zu einem Kalibriergas,

Fig. 6 ein Ausführungsbeispiel eines einfachen Aufbaus einer erfindungsgemässen Regelvorrichtung zur Brenngas-Sauerstoffträger-Verbundregelung einer Energiewandleranlage, und

Fig. 7 ein Ausführungsbeispiel einer Brennstoffzellenanlage mit Brenngas-Sauerstoffträger-Verbundregelung gemäss vorliegender Erfindung.

Fig. 7a ein anderes spezielles Ausführungsbeispiel gemäss Fig. 7.

Bei bekannter Wärmeleitfähigkeit $\kappa$ auf der linken Seite der Gleichung (2) kommt dem Volumen- bzw. Massenfluss $v_x$ bzw. $\rho \cdot v_x$, der Dichte $\rho$ und der Wärmekapazität $c_p$ im Vorfaktor

$$c_p \cdot \rho \cdot v_x , \qquad\qquad (5)$$

eine gleichwertige Rolle bei der Lösung der Gleichung (2) zu. Zur Bestimmung des Massenflusses ist also die Kenntnis der Wärmekapazität $c_p$ des Brenngases notwendig und für den Volumenfluss das Produkt $c_p \cdot \rho$. Da erfindungsgemäss derselbe Sensor sowohl zur Brenngasqualitäts- als auch zur Brenngasflussbestimmung benützt wird, stellt sich ein Brenngasflussfehler z.B. dann ein, wenn die Wärmekapazität $c_p$ des Brenngases oder das Produkt $c_p \cdot \rho$ in Gleichung (2) bzw. im Vorfaktor in Gleichung (5) nur ungenau bekannt ist.

Wird umgekehrt in einer Brenngas-Sauerstoffträger-Verbundregelung nur auf eine möglichst genaue Durchflussmessung geachtet und liegt keine Information über die Brenngasqualität vor, wie beispielsweise beim Gebrauch eines Ultraschalldurchflussmessers, wird zwar der Brenngasflussfehler $\Delta$ in Gleichung (3) zu Null, jedoch kann der sich einstellende Lambdawert $\lambda_{actual}$ wegen der Änderungen in $L_{min\_gas}$ stark variieren, was zur Russbildung in der Energiewandleranlage führen kann, wenn $\lambda_{actual}$ kleiner als der ideale Lambdawert $\lambda_{id}$ wird.

Fig. 2a zeigt die Korrelation zwischen idealem Verhältnis aus Sauerstoffträger- zu Brenngasmenge und der Wärmeleitfähigkeit des Brenngases für eine Brennstoffzellenanlage mit Hochtemperatur-Brennstoffzellen des SOFC-Typs.

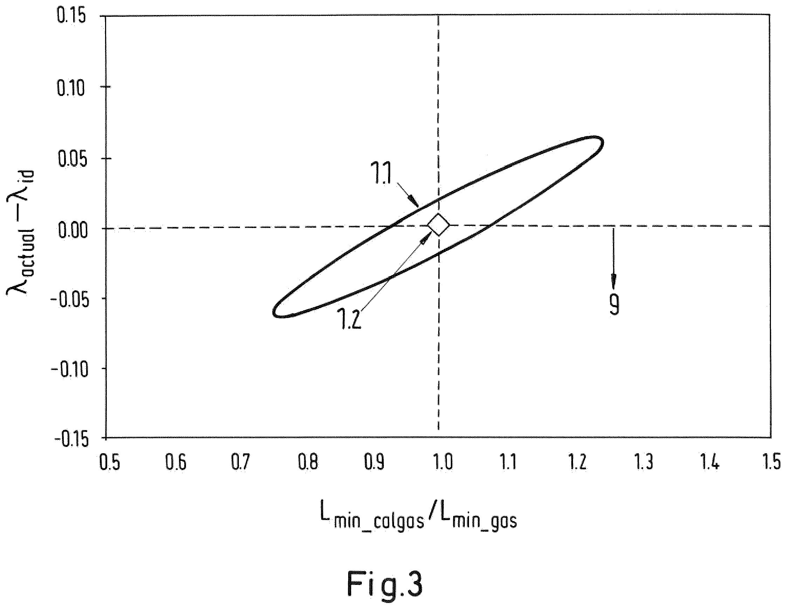

Fig. 3 zeigt die Abweichung $\lambda_{actual} - \lambda_{id}$ des sich einstellenden Lambdawertes zum idealen Lambdawert als Funktion des Sauerstoffträgerbedarfs $L_{min\_calgas} / L_{min\_gas}$ bei Unwissenheit der Brenngasqualität, aber fehlerloser Gasflussbestimmung im Falle einer SOFC-Brennstoffzelle mit partieller Oxidation im Vergleich zu einem Kalibriergas. Der Wert der Abweichung 1.2 für das Kalibriergas ist Null, während sich die Werte der Abweichung 1.1 für Erdgase für $\lambda_{actual} - \lambda_{id} < 0$ in das Gebiet 9 erstrecken, in dem Verrussungsgefahr besteht.

Handelsübliche, thermische "Massenflussmesser" bzw. "-regler" führen ihren Namen auf die Tatsache zurück, dass die Wärmeleitfähigkeit bei gegebenem Brenngas zwar unbekannt, aber bis auf eine Temperaturabhängigkeit konstant ist, was auch auf die Wärmekapazität zutrifft. In diesem Fall ist

$$\rho \cdot v_x, \qquad\qquad\qquad (6)$$

also der Massenfluss, die das System bestimmende Grösse, die sich dann durch Kalibrierung der unbekannten, konstanten Grössen $\kappa$ und $c_p$ eines Kalibriergases bestimmen lässt. Da der Heizdraht bei diesen Geräten entweder um die das Brenngas zuführende Messkapillare gewickelt ist oder, im Fall von direkt in den Brenngasstrom ragenden Heiz- und Fühlerelementen, eine Metallumhüllung aufweisen, ist beiden Varianten eine im Vergleich zum zugeführten Brenngas hohe thermische Wärmeleitung eigen, was den Einfluss des Brenngases vernachlässigbar macht. Gleichzeitig fällt damit aber auch die Möglichkeit weg, die Information der Wärmeleitfähigkeit eines unbekannten Brenngases zu benützen, um bei wechselnder Brenngasqualität bei der Brenngas-Sauerstoffträger-Verbundregelung entsprechende Korrekturen anzubringen. Der Einfluss der Wärmekapazität $c_p$ ist hingegen hauptsächlich auf das Brenngas zurückzuführen, was sich darin ausdrückt, dass Hersteller thermischer Massenflussmesser bzw. -regler für unterschiedliche Brenngase Konversionstabellen für den Fluss basierend auf der Wärmekapazität angeben (Vorfaktor in Gleichung (5)).

Anders verhält es sich beim Einsatz eines mikrothermischen Sensors, wie z.B. eines integrierten CMOS-Hitzdrahtanemometers. Zu dieser Technologie wird auf D. Matter, B. Kramer, T. Kleiner, B. Sabbattini, T. Suter, "Mikroelektronischer Haushaltsgaszähler mit neuer Technologie", Technisches Messen 71, 3 (2004), S. 137-146 hingewiesen.

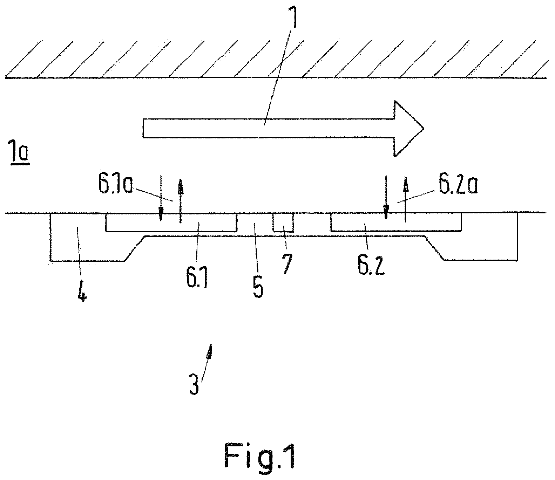

Ein Sensorkopf eines integrierten CMOS-Hitzdrahtanemometers ist in Fig. 1 gezeigt. Das CMOS-Hitzdrahtanemometer 3 aus Fig. 1 kann im Einsatz in einer Gasleitung 1a angeordnet und mit einem Brenngasstrom 1 oder fallweise auch mit einem Sauerstoffträgerstrom beaufschlag werden und umfasst ein Substrat 4, das typisch eine Membran 5 von wenigen Mikrometer Dicke enthält. Weiter umfasst das CMOS-Hitzdrahtanemometer zwei Thermoelement 6.1, 6.2 und ein Heizelement 7, das in Flussrichtung zwischen den beiden Thermoelementen angeordnet sein kann.

Die Methode mit mikrothermischem Sensor wie beispielsweise einem integrierten CMOS-Hitzdrahtanemometer unterscheidet sich von der Methode herkömmlicher, thermischer Massenflussmesser dadurch, dass der eigentliche Sensorkopf mit Heizelement 7 und Temperaturfühlerelementen 6.1, 6.2 auf einer nur wenigen Mikrometer dicken Membran 5 aufgebracht ist und direkt in den Brenngasstrom 1 ragt, wobei die Wärmeleitung der Membran in etwa vergleichbar ist mit derjenigen des Brenngases. Bei wechselnder Brenngasqualität wird deren Einfluss also spürbar, was es umgekehrt erlaubt, die Wärmeleitfähigkeit des Brenngases zu bestimmen. Mit der Möglichkeit der Wärmeleitfähigkeitsbestimmung kann mit der mikrothermischen Methode jegliches, thermisches Verfahren nachgeahmt werden. Es eröffnen sich aber noch weitere Möglichkeiten einer Brenngas-Sauerstoffträger-Verbundregelung, indem nebst der vollständigen Kompensation des Wärmeleitfähigkeitseinflusses eine nur teilweise oder sogar ausbleibende Kompensation letzterer ins Auge gefasst werden kann, wie im Folgenden dargestellt wird.

Mit einem integrierten, mikrothermischen Sensor ist z.B. die Abbildung der Funktionalität eines herkömmlichen, thermischen Massenflussmessers mit der alleinigen Bestimmung der Wärmeleitfähigkeit $\kappa$ in Gleichung (2) möglich, wenn man keine weitere Information über die Wärmekapazität $c_p$ als über diejenige des Kalibriergases einfliessen lässt. In Bezug auf den Lambdawert wirkt sich der sich einstellende Flussfehler in diesem Fall positiv auf das angestrebte Ziel möglichst kleiner, nur positiver Abweichung zum Idealwert aus (Gleichung (3)).

**EP 2 843 214 B1**

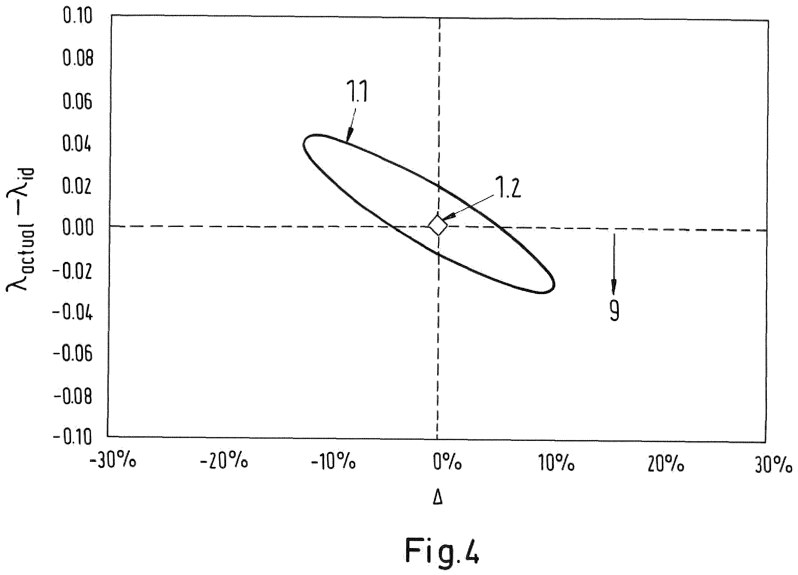

Fig. 4 zeigt die Abweichung $\lambda_{actual}$ - $\lambda_{id}$ des sich einstellenden Lambdawertes zum idealen Lambdawert als Funktion des Flussfehlers $\Delta$ für einen herkömmlichen Massenflussmesser bzw. -regler im Falle einer SOFC-Brennstoffzelle mit partieller Oxidation im Vergleich zu einem Kalibriergas. Der Wert der Abweichung 1.2 für das Kalibriergas ist Null, während sich die Werte der Abweichung 1.1 für Erdgase für $\lambda_{actual}$ - $\lambda_{id}$ < 0 in das Gebiet 9 erstrecken, in dem Verrussungsgefahr besteht.

Die Kenntnis der Wärmeleitfähigkeit $\kappa$ bringt eine weitere Verbesserung mit sich, da sich Informationen über $c_p$ eines unbekannten Brenngases gewinnen lassen, um eine Brenngasqualität unabhängige Massenflussmessung durchzuführen, was mit einem traditionellen Gerät nicht möglich ist.

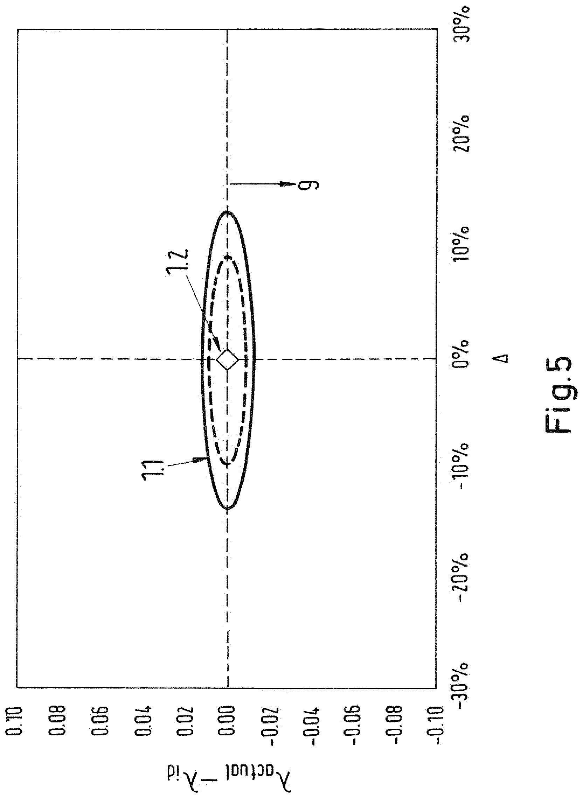

Fig. 5 zeigt die Abweichung $\lambda_{actual}$ - $\lambda_{id}$ des sich einstellenden Lambdawertes zum idealen Lambdawert als Funktion des Flussfehlers $\Delta$ für einen mikrothermischen Sensor im Falle einer SOFC-Brennstoffzelle mit partieller Oxidation im Vergleich zu einem Kalibriergas. Die mögliche Abweichung $\lambda_{actual}$ - $\lambda_{id}$ ist gegenüber der in Fig. 4 gezeigten Abweichung eines herkömmlichen Massenflussmeters deutlich geringer und die Werte der Abweichung 1.1 für Erdgase für $\lambda_{actual}$ - $\lambda_{id}$ < 0 erstrecken sich weniger weit in das Gebiet 9 so dass die Verrussungsgefahr vermindert wird.

Nimmt man die mit einem integrierten, mikrothermischen Sensor korrelierbaren Grössen wie z.B. den minimalen Sauerstoffträgerbedarf dazu (vergleiche Fig. 2), lassen sich die Werte der in Fig. 5 gezeigten Abweichung für Erdgase auf einen immer kleineren Bereich verkleinern (gestrichelte Ellipse), bis im Idealfall alle Brenngase auf den Fadenkreuzpunkt des Kalibriergases zu liegen kommen.

Mit anderen Worten wird die Aufgabe bezüglich des Verfahrens erfindungsgemäß gelöst durch eine mikrothermische Bestimmung des idealen Lambdawertes $\lambda_{id}$ und des minimalen Sauerstoffträgerbedarfs $L_{min}$ mit gleichzeitiger Bestimmung des Durchflusses, einerseits zur Erzielung des anwendungsspezifisch optimalen Prozesspunkts, andererseits zur Wahrung der Betriebssicherheit bei wechselnder Brenngasqualität, wobei das mikrothermische Verfahren die Möglichkeit bietet, je nach Anwendung einer Energiewandleranlage den Genauigkeitsgrad dieser Bestimmung und den damit verbunden Aufwand mit den entsprechenden Kosten zu wählen. Dies kann geschehen, indem nur ein Teil der relevanten Grundgrössen eines thermischen Verfahrens, d.h. Wärmeleitfähigkeit, Wärmekapazität, Massen- oder Volumenfluss betrachtet wird und die sich daraus ergebenden, fehlerkompensierenden Eigenschaften des Verfahrens ausgenutzt werden oder indem die genannten Grössen bestimmt und zur Korrelation von $\lambda_{id}$ und $L_{min}$ herangezogen werden.

Im erfindungsgemässen Verfahren werden somit der ideale Lambdawert, $\lambda_{id}$, und der minimale Sauerstoffträgerbedarf, $L_{min}$, in der Art und Weise bestimmt dass sich ein Lambdawert einstellt, für welchen die Differenz zum idealen Lambdawert des bei wechselnder Brenngasqualität zugrunde liegenden Brenngases einerseits zur Erzielung des anwendungsspezifisch optimalen Prozesspunkts möglichst klein, andererseits nie negativ wird, um die Betriebssicherheit nicht zu gefährden.

Gemäß einer vorteilhaften Ausführungsform der Erfindung werden mit dem mikrothermischen Verfahren die Grundgrössen Wärmeleitfähigkeit, Wärmekapazität, Massen- oder Volumenfluss bestimmt und $\lambda_{id}$ und $L_{min}$ daraus korreliert, was zu maximaler Effizienz und Betriebssicherheit des Systems führt.

Gemäß einer weiteren, vorteilhaften Ausführungsform der Erfindung wird mit dem mikrothermischen Verfahren der Einfluss der Grundgrössen Wärmeleitfähigkeit, Wärmekapazität, Massen- oder Volumenfluss nur teilweise kompensiert, wodurch sich ein gewisser Lambdawert $\lambda_{actual}$ einstellt, wobei sich der sich gleichzeitig einstellende Durchflussfehler dämpfend auf die Abweichung zum idealen Lambdawert $\lambda_{id}$ auswirkt.

Gemäß einer weiteren, vorteilhaften Ausführungsform der Erfindung wird mit dem mikrothermischen Verfahren der Einfluss der Grundgrössen Wärmeleitfähigkeit, Wärmekapazität, Massen- oder Volumenfluss nicht kompensiert, wodurch sich ein gewisser Lambdawert $\lambda_{actual}$ einstellt, wobei der sich gleichzeitig einstellende Durchflussfehler selbst in diesem Fall dämpfend auf die Abweichung zum idealen Lambdawert $\lambda_{id}$ auswirkt, was das Verfahren sehr kostengünstig macht.

Der erfindungsgemässe Sensor gemäss vorliegender Erfindung zur Brenngas-Sauerstoffträger-Verbundregelung einer gasbetriebenen Energiewandleranlage, ist mit einer Auswerte- und Regeleinheit ausgestattet, die zur Ausführung eines Verfahrens gemäß vorliegender Erfindung oder einer der oben beschriebenen Ausführungsformen desselben eingerichtet ist, und mit einem mikrothermischen Hitzdrahtanemometer, welches mit einem Brenngas beaufschlagbar ist, um mindestens zwei physikalische Kenngrössen des Brenngases zu ermitteln, zum Beispiel als erste physikalische Kenngrösse die Wärmeleitfähigkeit und/oder Wärmekapazität und als zweite physikalische Kenngrösse zum Beispiel den Massen- und/oder Volumenfluss eines mikrothermischen Verfahrens, und um aus den physikalischen Kenngrössen einen vom Brenngas oder von der Zusammensetzung des Brenngases abhängigen Sollwert für das Mischungsverhältnis zu bestimmen und damit die entsprechenden Brenngas- und/oder Sauerstoffträgermengen zu regeln.

Ein erfindungsgemäßer, mikrothermischer Sensor zur Brenngas-Sauerstoffträger-Verbundregelung einer Energiewandleranlage arbeitet nach dem erfindungsgemäßen Verfahren, beispielsweise mit einem Sensorblock mit integriertem, mikrothermischen CMOS-Hitzdrahtanemometer, über welches das Brenngas fliesst, durch Messen zweier Temperaturen stromauf- und stromabwärts zum Heizdraht, wie in Fig. 1 gezeigt. Die Temperaturinformation aus stromauf- und stromabwärts liegendem Temperaturfühler erlaubt es mit Hilfe der Lösung der Gleichung (2) die Grössen auf der rechten Seite in Gleichung (2) und separat die Wärmeleitfähigkeit des Brenngasgemisches zu bestimmen. Mit den derart bestimmten Grössen werden die Brenngas- und/oder Sauerstoffträgermengen geregelt.

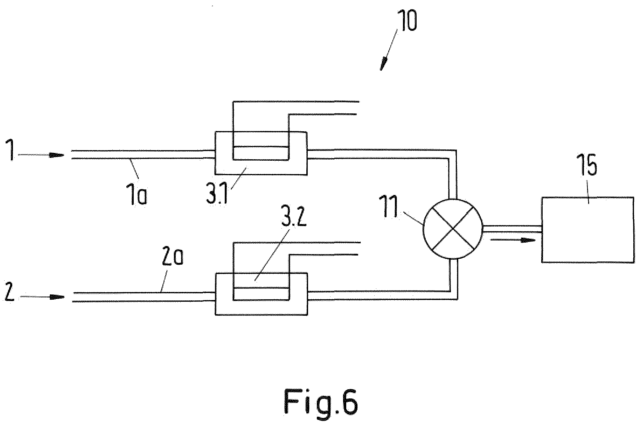

Ein Ausführungsbeispiel eines einfachen Aufbaus einer erfindungsgemässen Regelvorrichtung zur Brenngas-Sauerstoffträger-Verbundregelung einer Energiewandleranlage ist in Fig. 6 gezeigt. Die erfindungsgemässe Regelvorrichtung 10 zur Brenngas-Sauerstoffträger-Verbundregelung einer gasbetriebenen Energiewandleranlage enthält mindestens einen mikrothermischen Sensor 3.1, einen einfachen Flowsensor 3.2 zur Brenngas-Sauerstoffträger-Verbundregelung gemäß oben stehender Beschreibung und mindestens ein Ventil, insbesondere eine geregelte Mischeinrichtung 11 um die Brenngas- und/oder Sauerstoffträgermengen zu regeln.

In einem weiteren Ausführungsbeispiel der Regelvorrichtung fliessen Brenngas 1 aus der Gasleitung 1a und Sauerstoffträger 2 aus der Zuleitung 2a für den Sauerstoffträger zuerst jeweils durch einen Sensor 3.1 bzw. 3.2 mit integriertem CMOS-Hitzdrahtanemometer zur Messung von mindestens zwei physikalischen Kenngrössen, wobei die erste Kenngrösse insbesondere die Massen- und/oder Volumendurchflüsse und die zweite Kenngrösse zum Beispiel die Wärmeleitfähigkeit und/oder Wärmekapazität des Brenngases umfassen, woraus der ideale Lambdawert $\lambda_{id}$ und der minimale Sauerstoffträgerbedarf $L_{min}$ abgeleitet werden, um das richtige Mischverhältnis zwischen Brenngas und Sauerstoffträger einzuregeln. Brenngas und Sauerstoffträger können in einer Mischeinrichtung 11, insbesondere in einem Mischer 11 zusammengeführt und beispielsweise weiter zu einem gasbetriebenen Energiewandler 15 geleitet zu werden.

Die Erfindung umfasst zudem eine Brennstoffzellenanlage, insbesondere eine Brennstoffzellenanlage mit Hochtemperatur-Brennstoffzellen des SOFC-Typs, mit einer Regelvorrichtung zur Brenngas-Sauerstoffträger-Verbundregelung gemäss oben stehender Beschreibung.

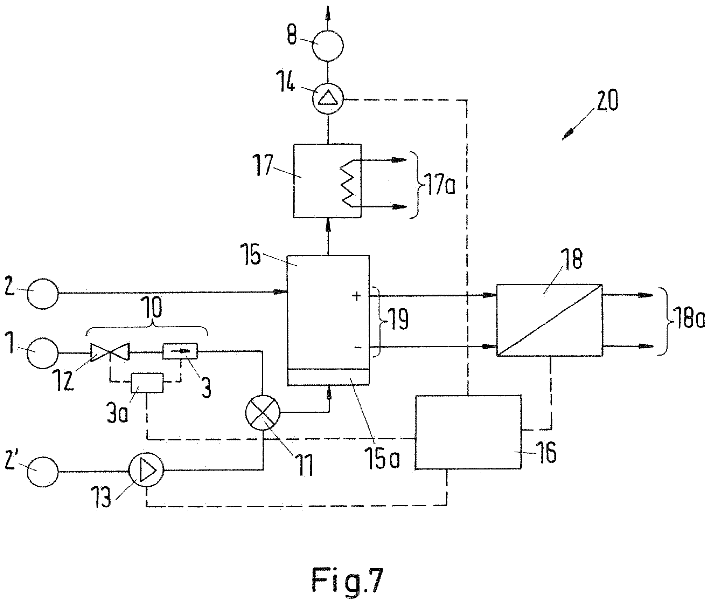

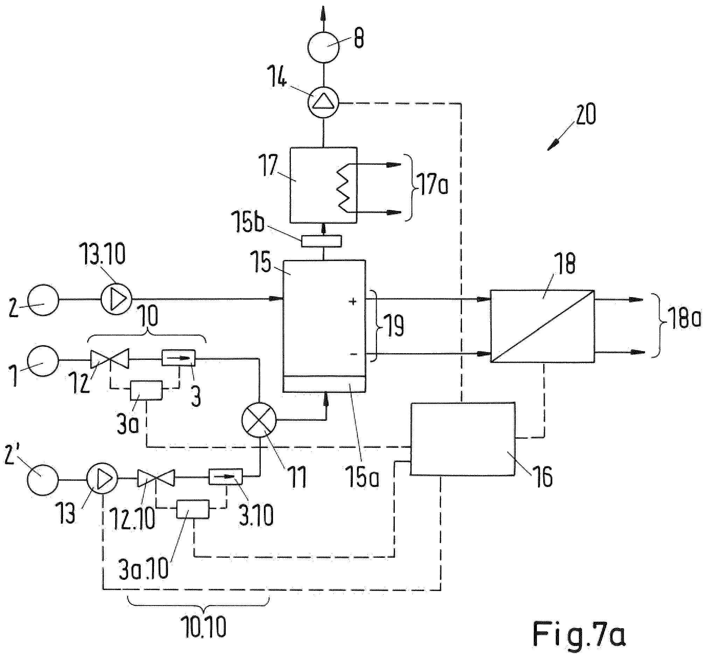

Ein Ausführungsbeispiel einer Brennstoffzellenanlage mit Brenngas-Sauerstoffträger-Verbundregelung gemäss vorliegender Erfindung wird in Fig. 7 gezeigt. Die Brennstoffzellenanlage enthält im Ausführungsbeispiel einen Brennstoffzellenstapel 15, der beispielsweise aus Hochtemperatur-Brennstoffzellen des SOFC-Typs ("Solid Oxid Fuel Cell") aufgebaut sein kann, die typisch bei einer Temperatur von 600°C bis 1000°C betrieben werden, und der die Nutzung von Energie eines Brennstoffs durch eine Energieumwandlung ermöglicht. Dabei können sowohl elektrische Energie, die aufgrund elektrochemischer Prozesse erzeugt wird, als auch thermische Energie, die in Form von heissen Abgasen der Prozesse anfällt, genutzt werden. Im Betrieb werden gasförmige Ströme eines Brenngases 1, im Fall von Erdgas eines reformierten Brenngases, und Sauerstoffträgers 2 getrennt durch die Zellen geführt. Der Sauerstoffträger 2 enthält insbesondere Umgebungsluft.

Normalerweise wird als Brenngas ein Methan enthaltendes Gas (z. B. Erdgas) verwendet, das vor dem Eintritt in die Zellen durch einen Reformer 15a geführt und dort beispielsweise, anders als in Fig. 7 auch endotherm unter Zufuhr von Prozesswärme in die reduzierenden Komponenten Wasserstoff und Kohlenmonoxid und weitere umgewandelt wird. Als Quelle für die im Reformer benötigte Prozesswärme in Form von Startwärme kann mit Vorteil das heisse Abgas verwendet werden.

Die Brennstoffzellenanlage enthält zusätzlich eine Regelvorrichtung 10 zur Brenngas-Sauerstoffträger-Verbundregelung, welche beispielsweise, wie in Fig. 7 gezeigt, in der Zuleitung für das Brenngas 1 angeordnet sein kann, um die Brenngasmenge und gegebenenfalls auch die Sauerstoffträgermenge zu steuern oder zu regeln. Im gezeigten Ausführungsbeispiel enthält die Regelvorrichtung mindestens einen mikrothermischen Sensor 3 gemäß oben stehender Beschreibung und mindestens ein Regelventil 12, wobei der mikrothermischen Sensor beispielsweise über eine Auswerte- und Regeleinheit 3a, die zur Ausführung eines Verfahrens gemäß vorliegender Erfindung oder einer der oben beschriebenen Ausführungsformen desselben eingerichtet ist, mit dem Regelventil verbunden ist.

Vorteilhafterweise enthält die Brennstoffzellenanlage zusätzliche eine Regelvorrichtung 10.10 zudem einen Massenflussmesser 3.10 und/oder einen Massenflussregler 3a.10 und/oder ein Regelventil 12.10, welche in der Zuleitung für den Sauerstoffträger 2' angeordnet sein können, um die Sauerstoffträgermenge zu steuern und/oder zu regeln. In dem in Fig. 7 bzw. Fig. 7a gezeigten Ausführungsbeispiel ist in der Zuleitung für den Sauerstoffträger 2' ein Gebläse 13 zur Druckerhöhung angeordnet, um die Sauerstoffträgermenge zu steuern und/oder zu regeln.

Weiter wird vorteilhafterweise nach der Brenngas-Sauerstoffträger Verbundgasregelung eine Mischvorrichtung 11 vorgesehen, um das Brenngas und den Sauerstoffträger zu mischen, bevor diese dem Reformer 15a zugeführt werden. Der benötigte Lambdawert des Brenngas-Sauerstoffträgergemisches hängt vom Reformprozess ab. Für einen typischen Reformprozess ohne Zuführung von Wasser liegt der benötigte Lambdawert zwischen 0.2 und 0.5, insbesondere zwischen 0.24 und 0.30.

Die Brennstoffzellen werden typisch bei einem Lambdawert zwischen 1.4 und 5, insbesondere zwischen 1.7 und 3 betrieben. Für die Zufuhr des hierzu benötigten Sauerstoffträgers 2 wird, wie in Fig. 7 gezeigt, eine separate Zuleitung zum Brennstoffzellenstapel vorgesehen sein.

Dem Brennstoffzellenstapel 15 nachgeschaltet ist in der Regel eine Nachverbrennungseinrichtung gefolgt von einem Wärmetauscher 17 verbunden, in dem den heissen Abgasen aus dem Brennstoffzellenstapel Wärme entzogen werden kann. Der Wärmetauscher 17 ist mit Vorteil mit einem Heizkreis 17a verbunden. Die Abgase 8 können anschliessend ins Freie geleitet werden oder der Restsauerstoff in den Abgasen kann in einem zusätzlichen Brenner, der in Fig.

7 nicht gezeigt ist, genutzt werden.

Bei Bedarf und in vorteilhafter Ausführung wird ausgangsseitig des Brennstoffzellenstapels 15 oder des Wärmetauschers 17 ein Abluftgebläse 14 angeordnet sein, mit welchem die Menge Sauerstoffträger durch den Brennstoffzellenstapel und damit wahlweise auch die Temperatur in den Brennstoffzellen gesteuert und/oder geregelt werden kann. Das Gebläse kann alternativ auch eintrittsseitig vom Brennstoffzellenstapel im Strang 2 (Gebläse 13.10) vorgesehen sein.

In einer vorteilhaften Ausführungsform enthält die Brennstoffzellenanlage 20 zudem einen Spannungswandler oder Wechselrichter 18, beispielsweise einen regelbaren Spannungswandler oder Wechselrichter, der mit dem Ausgang 19 des Brennstoffzellenstapels 15 verbunden ist. Vorteilhafterweise ist der Spannungswandler oder Wechselrichter mit einem Stromnetz 18a verbunden, um den im Brennstoffzellenstapel erzeugten Strom in das Stromnetz einzuspeisen.

In einer weiteren vorteilhaften Ausführungsform enthält die Brennstoffzellenanlage 20 zudem eine Steuereinheit 16, die wahlweise mit der Regelvorrichtung 10 und/oder dem Gebläse 13 und/oder dem Abluftgebläse 14 und/oder dem Spannungswandler oder Wechselrichter 18 verbunden sein kann.

Fig. 7a zeigt schliesslich ein anderes spezielles Ausführungsbeispiel, das sich von demjenigen der Fig. 7 nur dadurch unterscheidet, dass hier auch in der Zuleitung für den Sauerstoffträger 2' eine weitere Regelvorrichtung 10.10 mit Regelventil 12.10, Regeleinheit 3a.10, und Flow-Sensor 3.10, möglicherweise als mikrothermischer Sensor installiert ist, sowie in der Zuleitung für den Sauerstoffträger 2 zusätzlich als Option zum Abluftgebläse 14 noch ein Gebläse 13.10 vorgesehen ist.

Das Verfahren, der thermische Sensor und die Regelvorrichtung zur Brenngas-Sauerstoffträger-Verbundregelung gasbetriebener Energiewandleranlagen gemäss vorliegender Erfindung haben den Vorteil, dass die Differenz zwischen dem sich einstellenden Lambdawert und dem idealen Lambdawert des bei wechselnder Brenngasqualität zugrunde liegenden Brenngases vergleichsweise klein gehalten werden kann, was die Festlegung eines anwendungsspezifisch günstigen Betriebspunktes erlaubt. Gleichzeitig wird dank der Korrelation des idealen Lambdawertes $\lambda_{id}$ und des minimalen Sauerstoffträgerbedarfs $L_{min}$ mit den gemessenen physikalischen Kenngrössen ein sicherer Betrieb ermöglicht.

**Patentansprüche**

1. Verfahren zur Brenngas-Sauerstoffträger-Verbundregelung einer gasbetriebenen Energiewandleranlage (15, 20), wobei der Massen- oder Volumendurchfluss des Brenngases (1) und/oder Sauerstoffträgers (2) erfasst wird, um das Mischungsverhältnis (r) von Brenngas und Sauerstoffträger zu regeln, **dadurch gekennzeichnet, dass** mit einem mikrothermischen Sensor (3, 3.1, 3.2) mindestens zwei physikalische Kenngrössen des Brenngases ermittelt werden, wobei die erste Kenngrösse den Massen- und/oder Volumendurchfluss des Brenngases und die zweite Kenngrösse die Wärmeleitfähigkeit und/oder Wärmekapazität umfassen, und dass aus den physikalischen Kenngrössen ein vom Brenngas oder von der Zusammensetzung des Brenngases abhängiger Sollwert für das Mischungsverhältnis bestimmt wird, der zum Regeln des Mischungsverhältnisses verwendet wird, wobei der mikrothermische Sensor (3, 3.1, 3.2) ein mikrothermisches Hitzedrahtanemometer (3) ist, und die zwei physikalischen Kenngrössen, die mit demselben mikrothermischen Hitzedrahtanemometer (3) ermittelt wurden, sowohl zur Brenngasqualitäts- als auch zur Brenngasflussbestimmung benützt werden.

2. Verfahren nach Anspruch 1, wobei die zwei physikalischen Kenngrössen, die mit demselben mikrothermischen Sensor (3, 3.1, 3.2) ermittelt wurden, herangezogen werden, um einerseits mittels Korrelation den idealen Lambdawert ($\lambda_{id}$) und den minimalen Sauerstoffträgerbedarf ($L_{min}$) zu bestimmen und andererseits den Massen- und/oder Volumenfluss des Brenngases.

3. Verfahren nach einem der vorangehenden Ansprüche, wobei die Bestimmung der physikalischen Kenngrössen und der daraus korrelierten Grössen auf ein Kalibriergas bezogen wird, und wobei die Abweichung des kalibrierten Lambdawertes ($\lambda_{CalGas}$) zum idealen Lambdawert ($\lambda_{id}$) durch den damit gekoppelten Durchflussbestimmungsfehler ($\Delta$) teilweise oder ganz kompensiert wird.

4. Verfahren nach einem der vorangehenden Ansprüche, wobei die physikalischen Kenngrössen und die daraus korrelierten Grössen für ein beliebiges Brenngas in Teilen neu bestimmt und die restlichen vom Kalibriergas übernommen werden, und wobei die Abweichung des sich einstellenden Lambdawertes ($\lambda_{actual}$) zum idealen Lambdawert ($\lambda_{id}$) durch den damit gekoppelten Durchflussbestimmungsfehler ($\Delta$) zumindest teilweise kompensiert wird.

5. Verfahren nach Anspruch 4, wobei die Wärmekapazität und/oder Wärmeleitfähigkeit vom Kalibriergas übernommen werden.

6. Verfahren nach einem der Ansprüche 1 bis 3, wobei die physikalischen Kenngrössen und die daraus korrelierten Grössen für ein beliebiges Brenngas in Gänze vom Kalibriergas übernommen werden, und wobei die Abweichung des sich einstellenden Lambdawertes ($\lambda_{actual}$) zum idealen Lambdawert ($\lambda_{id}$) durch den damit gekoppelten Durchflussbestimmungsfehler ($\Delta$) zumindest teilweise kompensiert wird.

7. Verfahren nach einem der vorangehenden Ansprüche 1 bis 4, **dadurch gekennzeichnet, dass** die Energiewandleranlage (15, 20) eine Brennstoffzellenanlage, insbesondere Brennstoffzellenanlage mit Hochtemperatur-Brennstoffzellen des SOFC-Typs, eine Wärme-Kraft-Koppelungsanlage, ein Gasmotor, ein Heizgerät resp. eine Gastherme, eine gasbetriebene Wärmepumpe, ein Warmwassererzeuger, und/oder eine gasbetriebene Stromerzeugungsanlage oder eine Kombination davon ist.

8. Sensor, der ein mit einem Brenngas (1) beaufschlagbares mikrothermisches Hitzdrahtanemometer (3) und eine mit dem mikrothermischen Hitzdrahtanemometer (3) verbundene Auswerte- und Regeleinheit (3a) umfasst, wobei die Auswerte- und Regeleinheit (3a) zur Ausführung eines Verfahrens gemäss einem der Ansprüche 1 bis 7 eingerichtet ist; sowie zur Ermittlung von mindestens zwei physikalische Kenngrössen des Brenngases mit dem Hitzedrahtanemometer, wobei die erste Kenngrösse den Massen- und/oder Volumenfluss des Brenngases umfasst und die zweite Kenngrösse die Wärmeleitfähigkeit und/oder Wärmekapazität umfasst, eingerichtet ist; und eingerichtet ist, um aus den physikalischen Kenngrössen einen vom Brenngas oder von der Zusammensetzung des Brenngases abhängigen Sollwert für das Mischungsverhältnis zu bestimmen und damit die entsprechenden Brenngas- und/oder Sauerstoffträgermengen zu regeln.

9. Sensor nach Anspruch 8, wobei das eine mikrothermische Hitzdrahtanemometer, oder falls zwei mikrothermische Hitzdrahtanemometer verwendet werden, beide Hitzdrahtanemometer als integrierte CMOS-Hitzdrahtanemometer ausgeführt sind.

10. Sensor nach einem der Ansprüche 8 oder 9, wobei der Sensor (3, 3.1, 3.2) eingerichtet ist, um die Brenngasmenge und/oder Sauerstoffträgermenge mittels traditionellen thermischen Massenflussmessern bzw. -reglern zu regeln.

11. Regelvorrichtung zur Brenngas-Sauerstoffträger-Verbundregelung einer gasbetriebenen Energiewandleranlage, wobei die Regelvorrichtung (10) einen Sensor (3, 3.1) gemäß einem der Ansprüche 8 bis 10 und mindestens ein Regelventil (12), einen geregelten Lüfter (13) und / oder eine Regelvorrichtung (10.10) enthält, um die Brenngas- und/oder Sauerstoffträgermengen zu regeln.

12. Regelvorrichtung nach Anspruch 11, wobei die Regelventile (12, 12.10) als Mischventile bei der Mischeinrichtung (11) ausgebildet ist.

13. Brennstoffzellenanlage (20), insbesondere Brennstoffzellenanlage mit Hochtemperatur-Brennstoffzellen des SOFC-Typs, mit Regelvorrichtung (10, 10.10) zur Brenngas-Sauerstoffträger-Verbundregelung nach einem der Ansprüche 11 und 12.

**Claims**

1. A method for fuel gas - oxygen carrier combined control system of a gas-powered energy conversion system (15, 20), wherein the mass flow rate or volume flow rate of the fuel gas (1) and/or oxygen carrier (2) is detected in order to control the mixing ratio (r) of fuel gas and oxygen carrier, **characterized in that** at least two physical parameters of the fuel gas are determined with a microthermal sensor (3, 3.1, 3.2), wherein the first parameter comprises the mass flow rate and/or volume flow rate of the fuel gas and the second parameter comprises the heat conductivity and/or heat capacity, and **in that** a desired value for the mixing ratio which is dependent on the fuel gas or on the composition of the fuel gas is determined from the physical parameters and is used to control the mixing ratio, wherein the microthermal sensor (3, 3.1, 3.2) is a microthermal hot-wire anemometer (3), and the two physical parameters determined with the same microthermal hot-wire anemometer (3) are used for both fuel gas quality determination and fuel gas flow determination.

2. The method according to claim 1, wherein the two physical parameters determined with the same microthermal sensor (3, 3.1, 3.2) are used to determine, on the one hand, the ideal lambda value ($\lambda_{id}$) and the minimum oxygen carrier demand ($L_{min}$) by means of correlation and, on the other hand, the mass flow and/or volume flow of the fuel gas.

3. The method according to anyone of the preceding claims, wherein the determination of the physical parameters and the values correlated therefrom is referred to a calibration gas, and wherein the deviation of the calibrated lambda value ($\lambda_{CalGas}$) from the ideal lambda value ($\lambda_{id}$) is partially or completely compensated by the flow rate determination error ($\Delta$) coupled thereto.

4. The method according to anyone of the preceding claims, wherein the physical parameters and the values correlated therefrom are redetermined in parts for any fuel gas and the remaining ones are taken over from the calibration gas, and wherein the deviation of the resulting lambda value ($\lambda_{actual}$) from the ideal lambda value ($\lambda_{id}$) is at least partially compensated for by the flow rate determination error ($\Delta$) coupled thereto.

5. The method according to claim 4, wherein the heat capacity and/or the heat conductivity are taken over from the calibration gas.

6. The method according to anyone of the claims 1 to 3, wherein the physical parameters and the values correlated therefrom are taken over in their entirety from the calibration gas for any fuel gas, and wherein the deviation of the resulting lambda value ($\lambda_{actual}$) from the ideal lambda value ($\lambda_{id}$) is at least partially compensated for by the flow rate determination error ($\Delta$) coupled thereto.

7. The method according to anyone of the preceding claims 1 to 4, **characterized in that** the energy conversion system (15, 20) is a fuel cell system, in particular fuel cell system with high-temperature fuel cells of the SOFC type, a heat-power coupling system, a gas engine, a heater or a gas heater, a gas-powered heat pump, a hot water generator, and/or a gas-powered electricity generation system, or a combination thereof.

8. A sensor comprising a microthermal hot-wire anemometer (3) to which a fuel gas (1) can be applied and an evaluation unit and control unit (3a) connected to the microthermal hot-wire anemometer (3), wherein the evaluation unit and control unit (3a) is set up for carrying out a method according to anyone of the claims 1 to 7; and is set up for the determination of at least two physical parameters of the fuel gas with the hot-wire anemometer, wherein the first parameter comprises the mass flow and/or volume flow of the fuel gas and the second parameter comprises the heat conductivity and/or heat capacity; and is set up to determine a desired value for the mixing ratio which is dependent on the fuel gas or on the composition of the fuel gas from the physical parameters and thus to control the corresponding fuel gas amounts and/or oxygen carrier amounts.

9. The sensor according to claim 8, wherein the one microthermal hot-wire anemometer, or if two microthermal hot-wire anemometers are used, both hot-wire anemometers are designed as integrated CMOS hot-wire anemometers.

10. The sensor according to anyone of the claims 8 or 9, wherein the sensor (3, 3.1, 3.2) is set up to control the fuel gas amount and/or oxygen carrier amount by means of traditional thermal mass flow meters or mass flow controllers.

11. A control device for fuel gas-oxygen carrier combined control system of a gas-powered energy conversion system, wherein the control device (10) contains a sensor (3, 3.1) according to anyone of the claims 8 to 10 and at least one control valve (12), a controlled ventilator (13) and/or a control device (10.10) to control the fuel gas amounts and/or oxygen carrier amounts.

12. The control device according to claim 11, wherein the control valves (12, 12.10) are designed as mixing valves at the mixing device (11).

13. A fuel cell system (20), in particular fuel cell system with high-temperature fuel cells of the SOFC type, with control device (10, 10.10) for fuel gas-oxygen carrier combined control system according to anyone of the claims 11 and 12.

**Revendications**

1. Un procédé pour un système de réglage combiné gaz combustible-porteur d'oxygène d'un système de conversion d'énergie (15, 20) fonctionnant au gaz, dans lequel le débit massique ou le débit volumique du gaz combustible (1) et/ou du porteur d'oxygène (2) est détecté pour régler le rapport de mélange (r) du gaz combustible et du porteur d'oxygène, **caractérisé en ce qu'**au moins deux paramètres physiques du gaz combustible sont déterminés avec un capteur microthermique (3, 3.1, 3.2), dans lequel le premier paramètre comprend le débit massique et/ou le débit volumique du gaz combustible et le deuxième paramètre comprend la conductivité thermique et/ou la capacité thermique, et **en ce qu'**une valeur de consigne pour le rapport de mélange qui dépend du gaz combustible ou de la composition du gaz combustible est déterminée à partir des paramètres physiques et est utilisée pour régler le rapport de mélange, dans lequel le capteur microthermique (3, 3.1, 3.2) est un anémomètre à fil chaud microthermique (3), et les deux paramètres physiques déterminés avec le même anémomètre à fil chaud microthermique (3) sont utilisés à la fois pour la détermination de la qualité du gaz combustible et pour la détermination du débit de gaz combustible.

2. Le procédé selon la revendication 1, dans lequel les deux paramètres physiques déterminés avec le même capteur microthermique (3, 3.1, 3.2) sont utilisés pour déterminer, d'une part, la valeur lambda idéale ($\lambda_{id}$) et le besoin minimal en porteur d'oxygène ($L_{min}$) au moyen de corrélation, et, d'autre part, le débit massique et/ou le débit volumique du gaz combustible.

3. Le procédé selon l'une des revendications précédentes, dans lequel la détermination des paramètres physiques et des valeurs corrélées à ceux-ci est liée à un gaz d'étalonnage, et dans lequel la déviation de la valeur lambda étalonnée ($\lambda_{CalGas}$) par rapport à la valeur lambda idéale ($\lambda_{id}$) est partiellement ou totalement compensée par l'erreur de détermination du débit ($\Delta$) qui y est couplée.

4. Le procédé selon l'une des revendications précédentes, dans lequel les paramètres physiques et les valeurs corrélées à ceux-ci sont redéterminés en partie pour un gaz combustible quelconque et les autres sont reprises du gaz d'étalonnage, et dans lequel la déviation de la valeur lambda ($\lambda_{actual}$) qui se produit par rapport à la valeur lambda idéale ($\lambda_{id}$) est compensée au moins partiellement par l'erreur de détermination du débit ($\Delta$) qui y est couplée.

5. Le procédé selon la revendication 4, dans lequel la capacité thermique et/ou la conductivité thermique sont reprises du gaz d'étalonnage.

6. Le procédé selon l'une des revendications 1 à 3, dans lequel les paramètres physiques et les valeurs corrélées à ceux-ci sont reprises entièrement du gaz d'étalonnage pour un gaz combustible quelconque, et dans lequel la déviation de la valeur lambda ($\lambda_{actual}$) qui se produit par rapport à la valeur lambda idéale ($\lambda_{id}$) est compensée au moins partiellement par l'erreur de détermination du débit ($\Delta$) qui y est couplée.

7. Le procédé selon l'une des revendications précédentes 1 à 4, **caractérisé en ce que** le système de conversion d'énergie (15, 20) est un système de piles à combustible, en particulier un système de piles à combustible avec des piles à combustible à haute température du type SOFC, un système de couplage chaleur-force, un moteur à gaz, un appareil de chauffage ou un chauffage à gaz, une pompe à chaleur à gaz, un générateur d'eau chaude et/ou un système de production d'électricité à gaz, ou une combinaison de ceux-ci.

8. Un capteur qui comprend un anémomètre à fil chaud microthermique (3) pouvant être alimenté par un gaz combustible (1) et une unité d'évaluation et de réglage (3a) qui est reliée à l'anémomètre à fil chaud microthermique (3), dans lequel l'unité d'évaluation et de réglage (3a) est configurée pour exécuter un procédé selon l'une des revendications 1 à 7; et est configurée pour la détermination d'au moins deux paramètres physiques du gaz combustible avec l'anémomètre à fil chaud, dans lequel le premier paramètre comprend le débit massique et/ou le débit volumique du gaz combustible et le deuxième paramètre comprend la conductivité thermique et/ou la capacité thermique; et est configurée pour déterminer une valeur de consigne pour le rapport de mélange qui dépend du gaz combustible ou de la composition du gaz combustible à partir des paramètres physiques et pour régler ainsi les quantités correspondantes de gaz combustible et/ou de porteur d'oxygène.

9. Le capteur selon la revendication 8, dans lequel l'anémomètre à fil chaud microthermique, ou si deux anémomètres à fil chaud microthermique sont utilisés, les deux anémomètres à fil chaud sont conçus comme des anémomètres à fil chaud CMOS intégrés.

10. Le capteur selon l'une des revendications 8 ou 9, dans lequel le capteur (3, 3.1, 3.2) est configuré pour régler la quantité de gaz combustible et/ou la quantité de porteur d'oxygène au moyen de débitmètres ou de régulateurs massiques thermiques traditionnels.

11. Un dispositif de réglage pour un système de réglage combiné gaz combustible-porteur d'oxygène d'un système de conversion d'énergie fonctionnant au gaz, dans lequel le dispositif de réglage (10) contient un capteur (3, 3.1) selon l'une des revendications 8 à 10 et au moins une vanne de régulation (12), un ventilateur reglé (13) et/ou un dispositif de réglage (10.10) pour régler les quantités de gaz combustible et/ou les quantités de porteur d'oxygène.

**12.** Le dispositif de réglage selon la revendication 11, dans lequel les vannes de régulation (12, 12.10) sont conçues comme des vannes de mélange au niveau du dispositif de mélange (11).

**13.** Un système de piles à combustible (20), en particulier système de piles à combustible avec des piles à combustible à haute température du type SOFC, avec un dispositif de réglage (10, 10.10) pour un système de réglage combiné gaz combustible-porteur d'oxygène selon l'une des revendications 11 et 12.

Fig.1

Fig.2

EP 2 843 214 B1

Fig.2a

Fig.3

Fig.4

Fig.5

Fig.6

Fig.7

Fig.7a

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2006061228 A1
- EP 1923634 A1
- EP 2574918 A1

**In der Beschreibung aufgeführte Nicht-Patentliteratur**

- **KERSON HUANG.** Statistical Mechanics. John Wiley & Sons, 1987
- **D. MATTER ; B. KRAMER ; T. KLEINER ; B. SABBATTINI ; T. SUTER.** Mikroelektronischer Haushaltsgaszähler mit neuer Technologie. *Technisches Messen,* 2004, vol. 71 (3), 137-146